# EUROPEAN PATENT SPECIFICATION

(11) **EP 1 874 812 B1**
(45) Date of publication and mention of the grant of the patent: **04.09.2013**
(21) Application number: 06751224.4
(22) Date of filing: 24.04.2006
(51) Int. Cl.: C07K 14/52, C07K 14/54, C07K 14/705, C07K 14/73

(54) **METHODS OF ASSESSING ALLERGENICITY OF PROTEASES**
METHODEN ZUR BESTIMMUNG DER ALLERGENITÄT VON PROTEASEN
PROCEDURES D'ÉVALUATION D'ALLERGÉNICITÉ DES PROTEASES

(30) Priority: 27.04.2005 US 675156 P
(43) Date of publication of application: 09.01.2008
(73) Proprietor: Danisco US Inc., Palo Alto, California 94304 (US)
(72) Inventor: HARDING, Fiona A., Genencor International, Inc., Palo Alto, California 94304 (US)
(74) Representative: Forrest, Graham Robert
(86) International application number: PCT/US2006/015441
(87) International publication number: WO 2006/116292

(56) References cited:
- WO-A-00/48462
- WO-A-02/077187
- WO-A-03/073068
- WO-A-03/104263
- US-B1- 6 232 088
- WOOD P.A., KAATTARI S.L.: "Enhanced immunogenicity of Renibacterium salmoninarium in chinook salmon after removal of teh bacterial cell surface-associated 57 kDa protein" DISEASES OF AQUATIC ORGANISMS, vol. 25, 1996, pages 71-79, XP009073486
- STEWART G A ET AL: "Endogenous function and biological significance of aeroallergens: an update" CURR OPIN ALLERGY CLIN IMMUNOL, vol. 1, no. 1, 2001, pages 95-103, XP009074622
- WIDMER F ET AL: "Substrate preference profiles of proteases released by allergenic pollens" CLINICAL AND EXPERIMENTAL ALLERGY, vol. 30, 2000, pages 571-576, XP002406155
- MURALI R. ET AL: "Altered levels of urokinase on monocytes and in serum of children with AIDS; effects on lymphocyte activation and surface marker expression" JOURNAL OF LEUKOCYTE BIOLOGY, vol. 64, 1998, pages 198-202, XP000993187
- WESTON G S ET AL: "GRANZYMES AS POTENTIAL TARGETS FOR RATIONAL DRUG DESIGN" CURRENT MEDICINAL CHEMISTRY, BENTHAM SCIENCE PUBLISHERS BV, BE, vol. 3, no. 1, 1996, pages 37-46, XP002913206 ISSN: 0929-8673
- SHAKIB F.,GOUGH L.: "The proteolytic activity of Der p 1 selectively enhances IgE synthesis: a link between allerneicity and cyteine protease activity" CLINICAL AND EXPERIMENTAL ALLERGY, vol. 30, 2000, pages 751-752, XP002402426 cited in the application
- CHEUNSUK S.: "Analysis of the IDDM Candidate Gene Prss16 in NOD and NON Mice" DEVELOPMENTAL IMMUNOLOGY, vol. 9, no. 4, 2002, pages 183-186, XP009074570
- XUE A ET AL: "HLA-DQ8 is a predisposing molecule for detergent enzyme subtilisin BPN'-induced hypersensitivity" CLINICAL IMMUNOLOGY, vol. 117, 2005, pages 302-315, XP002406156

## Description

### FIELD OF THE INVENTION

The present invention provides means to assess the comparative allergenicity of proteases. In particular, the present invention provides means to qualitatively assess the potential for any protease to induce an allergic response in humans. In addition, the present invention provides means to select proteases with reduced allergenicity for use in various applications.

### BACKGROUND OF THE INVENTION

Proteins, including proteases, have the capacity to induce potentially life-threatening immune responses. This limitation has hindered their widespread use in consumer end-use applications and products. Indeed, this potential to induce immune responses has come to the attention of the U.S. Food and Drug Administration (FDA), resulting in the requirement for allergenicity testing both prior to and after approval of new protein therapeutics. However, although there are a number of animal models available for assessing allergenicity, there are no validated methods to discern relative allergenicity in humans.

Indeed, the allergenicity of proteins, including proteases, has long been a concern in the enzyme manufacturing industry. Occupational exposure to proteins (*e.g*., proteases) has been documented to result in sensitization of industrial and laboratory workers. Sensitization to particular proteins is usually assessed by tests (*e.g*., the skin-prick test) that reveal whether an individual has mounted an immune response to the protein.

The manufacture of industrial proteases has been shown to induce sensitization in exposed workers (*See e.g.,* Novey et al., J. Allergy Clin. Immunol., 63:98 [1979]; Pepys et al., Clin. Allergy 15:101 [1985]; Vanhanen et al., Occup. Environ. Med., 57:121 [2000]; Schweigert et al., Clin. Exp. Allergy 30:1511 [2000]; and Sarlo et al., Fund. Appl. Toxicol., 39:44 [1997]). The occupational exposure guideline set by the American College of American Government Industrial Hygienists is currently at 60 ng/m³ (Groux et al., Nature 389:737 [1997]). Implementation of work practices and control measures has reduced the number of allergy cases among occupationally exposed workers (*See e.g.,* Sarlo, Ann. Allergy Asthma Immunol., 90:32 [2003]; and Yamagiwa et al., J. Immunol., 166:7282 [2001]). The rates of sensitization have been reported to range from 3 to 11 percent per year, depending on the material being processed (*See e.g.,* Sarlo, Ann. Allergy Asthma Immunol., 90:32 [2003]; and Robinson et al., Toxicol. Sci., 43:39 [1998]). Two highly related proteases, *B. lentus* subtilisin and BPN' Y217L, are known to induce distinctly different rates of sensitization in exposed industrial workers. However, the quantities of protease encountered during occupational exposure are inconsistent with the level of enzyme necessary *in vitro* and *in vivo* to exert effects on cell surface markers and to initiate Th2 responses. Thus, much remains unknown regarding the factors that involve T-cell differentiation, particularly Th differentiation and any relationship with between this process and proteolytic activity.

The activation of CD4+ T-cells along their differentiation pathways results from antigen-specific signaling by antigen-presenting cells. The factors that control the differentiative outcome of antigen signaling are complex, but include cytokine-mediated signaling. WO01/29562 describes in vitro methods for assessing the allergenicity of a test compound by exposing the test compound to a culture of immune cells and determining the changes in cytokine profile in the cell culture in response to the test compound. The cytokines IL-4 and IL-12 are involved in instructing naïve CD4+ T-cells to become Th2 and Th1 cells, respectively. IL-4 is an absolute requirement for the development of Th2 responses (Le Gros et al., J. Exp. Med., 172:921 [1990]). Another outcome of T-cell activation is the induction of non-responsiveness. There are a number of mechanisms whereby tolerance to antigens is established, including deletional mechanisms that are active both in the thymus during development and in the periphery in response to exogenous antigen; clonal "ignorance" in which T-cells with specificity for antigen do not respond to sequestered antigens; and active suppression of responses by regulatory T-cells. The differentiation of regulatory T-cells may also be influenced by cytokine signaling, as TGF-beta and IL-10 have been shown to be necessary for their induction (*See e.g.,* Chen et al., J. Exp. Med., 198:1875 [2003]; and Levings et al., Blood, 105:1162 [2005]). IgE-mediated hypersensitivity reactions are a result of a subset of immune responses characterized by the preferential differentiation of Th2 CD4+ T-cells. The induction of allergic responses tends to occur at mucosal surfaces (*e.g*., in the lungs and gut). Regulatory T-cells in the lungs and the gut are believed to control allergic type responses in non-allergic individuals. Thus, disregulation of antigen-specific responses results in allergy. The properties of allergens that sway immune responses from the normal non-allergic responses to commonly encountered proteins to Th2-type responses are not completely understood. However, many allergens, including many respiratory allergens, have been found to possess proteolytic activity. For example, pollen allergens are a complex mixture of many proteins including a variety of peptidases. Also, many recombinant allergen proteins also display proteolytic activity (*See e.g.,* Hewitt et al., Allergy 53:60 [1998]; and Bagarozzi et al., Phytochem., 47:593 [1998]).

The contribution of proteolytic activity to allergenic potency has been investigated. Proteolytic activity was found to be necessary to induce allergic responses, as inactivated proteases were not allergenic (*See e.g.,* Shakib and Gough, Clin. Exp. Allergy 30:751 [2000]; Pollock et al., J. Immunol., 170:1746 [2003]; and Kheradmand et al., J. Immunol., 169:5904 [2002]). In some experiments, it was determined that co-administration of a proteolytic allergen in conjunction with a normally non-allergenic protein, induced IgE responses to the second protein, demonstrating that the proteolytic activity was acting in trans. The mechanism by which the proteolytic activity affected the immune response was described as being contributed by an effect on cell-surface regulatory molecules. Based on these and other reports, removal of CD23 and CD25 from the surface of responding cells provides a very satisfactory explanation for the proteolytic activity on the immune response. Nonetheless, methods currently used in the art fail to sufficiently address the contribution(s) of protease activity on allergenicity of proteins.

### SUMMARY OF THE INVENTION

The present invention provides means to assess the comparative allergenicity of proteases. In particular, the present invention provides means to qualitatively assess the potential for any protease to induce an allergic response in humans. In addition, the present invention provides means to select proteases with reduced allergenicity for use in various applications.

Thus, the invention provides an in vitro method for assessing the potential of a protease to induce an allergic response in humans comprising exposing a cytokine selected from the group consisting of interleukins, interferons and transforming growth factors to said protease, and comparing the activity of said cytokine before and after exposure to said protease.

The cytokine may be a human cytokine, or may be from other animals. It may be selected from the group consisting of IL-4, IL-13, IL-12, TGF-β and IL-10.

The protease may be a serine protease. The serine protease may be a subtilisin, e.g. *B. lentus* subtilisin or *B. amyloliquefaciens* subtilisin (BPN'), or variants of said *B*. *lentus* or *B. amyloliquefaciens* subtilisins such as BPN' Y217L.

### BRIEF DESCRIPTION OF THE FIGURES

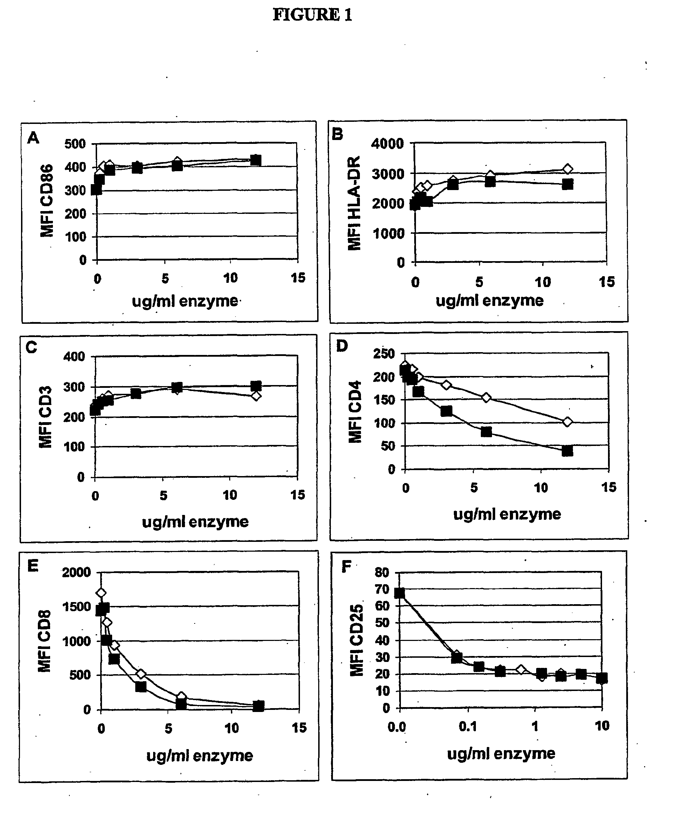

Figure 1 provides graphs showing enzymatic activity on the expression of cell surface molecules. Human peripheral blood mononuclear cells were treated with the indicated concentrations of enzyme in PBS at RT for 60 minutes. The reaction was stopped with 2% FCS.
BPN' Y217L is indicated by closed squares, while subtilisin is indicated by open diamonds. In Panels A and B, PBMC were gated for monocytes using FSC/SSC parameters, while in Panels C-E, PBMC were gated on lymphocytes using FSC/SSC parameters. Panel F shows the results for PHA blasts treated with enzymes for 30 minutes, with gating on FSC/SSC. Panel A provides results for CD86, while Panel B provides results for HLA-DR, Panel C provides results for CD3, Panel D provides results for CD4, Panel E provides results for CD8, and Panel F provides results for CD25.

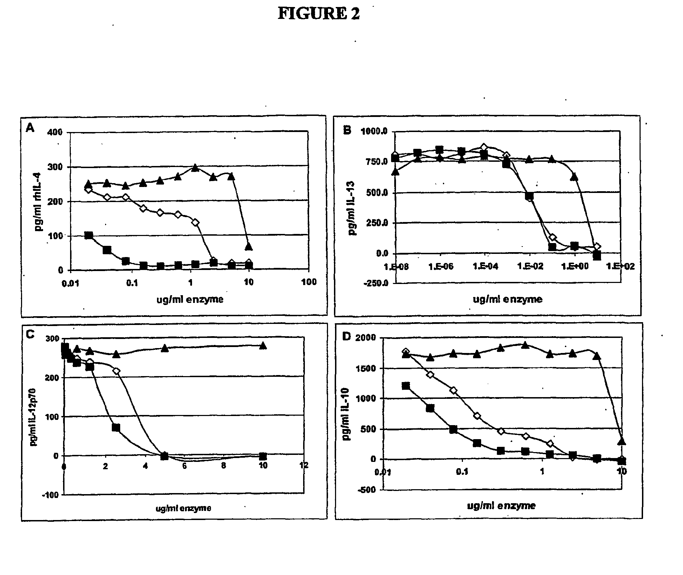

Figure 2 provides graphs showing that BPN' Y217L and *B. lentus* subtilisin have different proteolytic activity on human cytokines. In these graphs, BPN' Y217L (squares), subtilisin (diamonds), or N155G (triangles) were incubated at 37°C for 18 hrs, in 5% human serum with the indicated cytokines (Panel A provides results for IL-4, Panel B provides results for IL-13, Panel C provides results for IL-12p70, and Panel D provides results for IL-10). Residual cytokine levels were measured using ELISA assays.

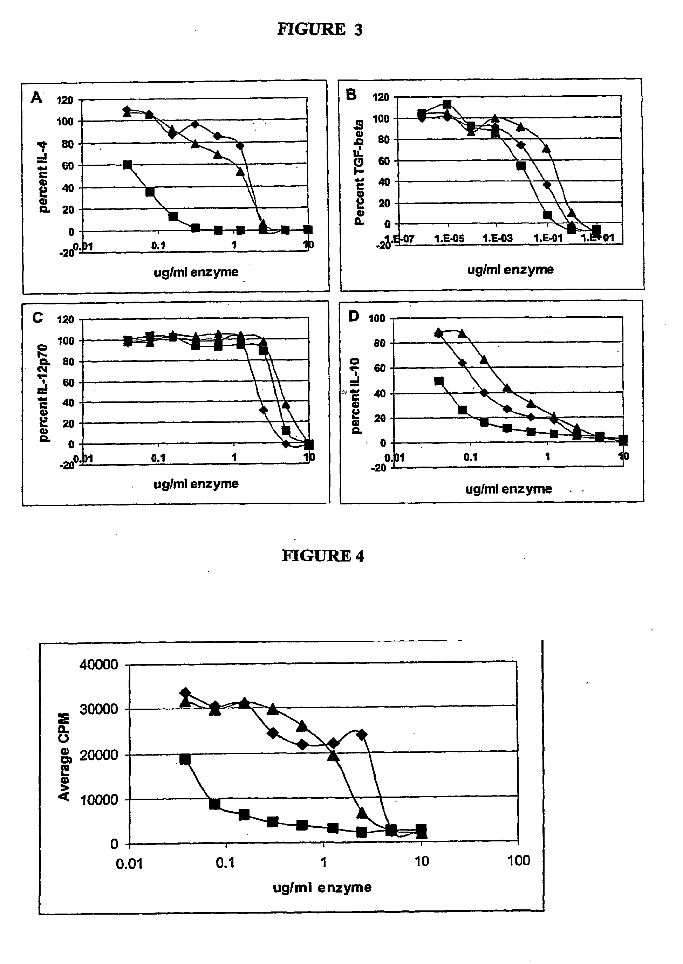

Figure 3 provides graphs showing that *B. licheniformis* Carlsberg and *B. lentus* subtilisin exhibit similar proteolytic activity on IL-4, IL-10, IL-12 and TGF-beta. BPN' Y217L (squares), *B. lentus* subtilisin (diamonds) or *B. licheniformis* Carlsberg enzyme (triangles) were incubated at 37°C for 18 hrs in 5% human serum with the indicated cytokines (Panel A provides results for IL-4, while Panel B provides results for TGF-β, Panel C provides results for IL-12p70, and Panel D provides results for IL-10). Residual cytokine was measured in ELISA assays.

Figure 4 provides a graph showing that enzyme activity reduces functional activity of IL-4. In these experiments, 500 ng/ml human IL-4 was treated with *B. lentus* subtilisin (diamonds), BPN' Y217L (squares) or *B. licheniformis* Carlsberg (triangles) at the indicated concentrations overnight at 37°C. Enzymes were inactivated by the addition of PMSF, then 2 x 10⁴ TF-1 cells were added per well. Proliferation was determined on day 3. Untreated control cultures contained 500 ng/ml IL-4 and displayed an average of 31099 CPM.

### DESCRIPTION OF THE INVENTION

The present invention provides means to assess the comparative allergenicity of proteases. In particular, the present invention provides means to qualitatively assess the potential for any protease to induce an allergic response in humans. In addition, the present invention provides means to select proteases with reduced allergenicity for use in various applications.

### Definitions

Unless defined otherwise herein, all technical and scientific terms used herein have the same meaning as commonly understood by one of ordinary skill in the art to which this invention pertains. For example, Singleton and Sainsbury, Dictionary of Microbiology and Molecular Biology, 2d Ed., John Wiley and Sons, NY (1994); and Hale and Marham, The Harper Collins Dictionary of Biology, Harper Perennial, NY (1991) provide those of skill in the art with a general dictionaries of many of the terms used in herein. Although any methods and materials similar or equivalent to those described herein find use in the practice of the present invention, the preferred methods and materials are described herein. Accordingly, the terms defined immediately below are more fully described by reference to the Specification as a whole.

As used herein, the term "immune response" refers to the immunological response mounted by an organism (*e.g.,* a human or other animal) against an immunogen. It is intended that the term encompass all types of immune responses, including but not limited to humoral (*i.e*., antibody-mediated), cellular, and non-specific immune responses. In some embodiments, the term reflects the immunity levels of populations (*i.e*., the number of people who are "immune" to a particular antigen and/or the number of people who are "not immune" to a particular antigen).

As used herein, the term "reduced immunogenicity" refers to a reduction in the immune response that is observed with variant (*e.g*., derivative) proteins, as compared to the original wild-type (*e.g*. parental or source) proteins. In preferred embodiments of the present invention, variant proteins that stimulate a less robust immune response *in vitro* and/or *in vivo,* as compared to the source protein are provided. It is contemplated that these proteins having reduced immunogenicity will find use in various applications, including but not limited to bioproducts, protein therapeutics, food and feed, personal care, detergents, and other consumer-associated products, as well as in other treatment regimens, diagnostics, etc.

As used herein, the term "enhanced immunogenicity" refers to an increase in the immune response that is observed with variant (*e.g*., derivative) proteins, as compared to the original wild-type (*e.g*. parental or source) proteins. In preferred embodiments of the present invention, variant proteins that stimulate a more robust immune response *in vitro* and/or i*n vivo,* as compared to the source protein are provided. It is contemplated that these proteins having enhanced immunogenicity will find use in various applications, including but not limited to vaccines, bioproducts, therapeutics, food and feed additives, as well as in other treatment regimens, diagnostics, etc.

As used herein, the term "reduced allergenicity" refers to a reduction in the allergic immune response that is observed with variant (*e.g.*, derivative) proteins, as compared to the original wild-type (*e.g*. parental or source) proteins. In preferred embodiments of the present invention, variant proteins that stimulate a less robust allergic response in *vitro* and/or *in vivo,* as compared to the source protein are provided. It is contemplated that these proteins having reduced allergenicity will find use in various applications, including but not limited to bioproducts, protease therapeutics, food and feed, personal care, detergents, and other consumer-associated products, as well as in other treatment regimens, diagnostics, etc.

The term "sample" as used herein is used in its broadest sense. However, in preferred embodiments, the term is used in reference to a sample (*e.g*., an aliquot) that comprises a composition of interest that is being analyzed, identified, modified, and/or compared with other compositions

As used herein, the terms "T lymphocyte" and "T-cell," encompass any cell within the T-lymphocyte lineage from T-cell precursors (including Thyl positive cells which have not rearranged the T-cell receptor genes) to mature T-cells (*i.e*., single positive for either CD4 or CD8, surface TCR positive cells).

As used herein, "cell surface markers" refers to molecules ("markers") that are expressed on the surface of cells of a particular type. For example, T-cells express various "CD" ("cluster designation") markers on their surfaces, including but not limited to CD3, CD4, CD8, CD25, CD86, etc. Expression of certain CDs is characteristic of particular cell types. Thus, the expression of various molecules on the cell surface finds use in distinguishing cell subsets from each other.

As used herein, "removal" of cell surface markers refers to the use of protease to effect the removal of cell surface molecules, such that fewer markers are expressed on the surface of the cells treated with the protease. It is not intended that the protease treatment completely remove all of the cell surface markers, although it is contemplated that this will occur in some cases and/or under certain conditions.

As used herein, "protein" refers to any composition comprised of amino acids and recognized as a protein by those of skill in the art. The terms "protein," "peptide" and polypeptide are used interchangeably herein. Wherein a peptide is a portion of a protease, those skill in the art understand the use of the term in context.

As used herein, "protein of interest" refers to a protein (*e.g*., a protease) which is being analyzed, identified and/or modified. Naturally-occurring, as well as recombinant proteins find use in the present invention. Indeed, the present invention finds use with any protein for which it is desired to characterize and/or modulate the immunogenic response of humans (and/or other animals). In some embodiments, proteins including hormones, cytokines, antibodies, enzymes, structural proteins and binding proteins find use in the present invention. In some particular embodiments, the "protein of interest" is a "cytokine of interest."

As used herein, "cytokine" refers to the soluble mediators that control many critical interactions among cells of the immune system. Cytokines comprise a diverse group of intercellular signaling peptides and glycoproteins. Most are genetically and structurally similar to each other. Each cytokine is secreted by a particular cell type in response to a variety of stimuli and produces characteristic effects on the growth, mobility, differentiation, and/or function of target cells. Collectively, cytokines regulate not only immune and inflammatory systems, but also are involved in wound healing, hematopoiesis, angiogenesis, and many other processes. It is intended that the term encompass all of the various cytokines, regardless of their structure, and commonly used nomenclature. For example, it is intended that the term encompass "lymphokines" (*i.e*., cytokines produced by lymphocytes), as well as "monokines" (*i.e*., cytokines produced by monocytes).

As used herein, "cytokine receptor" refers to receptor molecules that recognize and bind to cytokines. It is intended that the term encompass soluble cytokine receptors as well as cytokine receptors that are cell-bound. It is intended that the term also encompass modified cytokine receptor molecules (*i.e*., "variant cytokine receptors"), including those with substitutions, deletions, and/or additions to the cytokine receptor amino acid and/or nucleic acid sequence. Thus, it is intended that the term encompass wild-type, as well as recombinant, synthetically-produced, and variant cytokine receptors.

As used herein, "wild-type" and "native" proteins are those found in nature. The terms "wild-type sequence," and "wild-type gene" are used interchangeably herein, to refer to a sequence that is native or naturally occurring in a host cell. In some embodiments, the wild-type sequence refers to a sequence of interest that is the starting point of a protein engineering project.

As used herein, "protease" refers to naturally-occurring proteases, as well as recombinant proteases. The term "protease" encompasses mature forms of proteases, as well as the pro- and prepro-forms of related proteases. Prepro forms of proteases comprise the mature form of the protease having a prosequence operably linked to the amino terminus of the protease, and a "pre-" or "signal" sequence operably linked to the amino terminus of the prosequence. Proteases are enzymes which generally act to cleave peptide bonds of proteases or peptides. Naturally-occurring proteases include, but are not limited to such examples as α-aminoacylpeptide hydrolase, peptidylamino acid hydrolase, acylamino hydrolase, serine carboxypeptidase, metallocarboxypeptidase, thiol proteases, carboxylproteases and metalloproteases. Serine, metallo, thiol and acid proteases are included, as well as endo and exo-proteases. Indeed, in some preferred embodiments, serine proteases such as chymotrypsin and subtilisin find use. Chymotrypsin and subtilisin have a catalytic triad comprising aspartate, histidine and serine. In the subtilisin proteases, the relative order of these amino acids reading from the carboxy terminus is aspartate-histidine-serine, while in the chymotrypsin proteases, the relative order of these amino acids reading from the carboxy terminus is histidine-aspartate-serine. Although subtilisins are typically obtained from bacterial, fungal or yeast sources, "subtilisin" as used herein, refers to a serine protease having the catalytic triad of the subtilisin proteases defined above. Additionally, human subtilisins are proteases of human origin having subtilisin catalytic activity, for example the kexin family of human derived proteases. Subtilisins are well known by those skilled in the art for example, *Bacillus amyloliquefaciens* subtilisin (BPN'), *Bacillus lentus* subtilisin (*e.g.*, SAVINASE® subtilisin), *Bacillus subtilis* subtilisin, *Bacillus licheniformis* subtilisin (*See e.g.,* U.S. Patent 4,760,025 (RE 34,606), U.S. Patent 5,204,015, U.S. Patent 5,185,258, EP 0 328 299, and WO89/06279).

As used herein, functionally similar proteases are considered to be "related proteases." In some embodiments, these proteases are derived from a different genus and/or species (*e.g., B. subtilis* subtilisin and *B. lentus* subtilisin), including differences between classes of organisms (*e.g*., a bacterial subtilisin and a fungal subtilisin). In additional embodiments, related proteases are provided from the same species. Indeed, it is not intended that the present invention be limited to related proteases from any source(s).

As used herein, the term "derivative" refers to a protein (*e.g.,* a protease) which is derived from a precursor protein (*e.g.*, the native protease) by addition of one or more amino acids to either or both the C- and N-terminal end(s), substitution of one or more amino acids at one or a number of different sites in the amino acid sequence, and/or deletion of one or more amino acids at either or both ends of the protein or at one or more sites in the amino acid sequence, and/or insertion of one or more amino acids at one or more sites in the amino acid sequence. The preparation of a protein derivative is preferably achieved by modifying a DNA sequence which encodes for the native protein, transformation of that DNA sequence into a suitable host, and expression of the modified DNA sequence to form the derivative protein.

One type of related (and derivative) proteases is "variant proteases." In preferred embodiments, variant proteases differ from a parent protease and one another by a small number of amino acid residues. The number of differing amino acid residues may be one or more, preferably 1, 2, 3, 4, 5, 10, 15, 20, 30, 40, 50, or more amino acid residues. In one preferred embodiment, the number of different amino acids between variants is between 1 and 10. In particularly preferred embodiments, related proteases and particularly variant proteases comprise at least 50%, 60%, 65%. 70%, 75%, 80%, 85%, 90%, 95%, 97%, 98%, or 99% amino acid sequence identity. Additionally, a related protease or a variant protease as used herein, refers to a protease that differs from another related protease or a parent protease in the number of prominent regions. For example, in some embodiments, variant proteases have 1, 2, 3, 4, 5, or 10 corresponding prominent regions which differ from the parent protease. In one embodiment, the prominent corresponding region of a variant produces only a background level of immunogenic response.

As used herein, "corresponding to," refers to a residue at the enumerated position in a protease or peptide, or a residue that is analogous, homologous, or equivalent to an enumerated residue in another protease or peptide.

As used herein, "corresponding region" generally refers to an analogous position within related proteases or a parent protease.

As used herein, the term "analogous sequence" refers to a sequence within a protein that provides similar function, tertiary structure, and/or conserved residues as the protein of interest. In particularly preferred embodiments, the analogous sequence involves sequence(s) at or near an epitope. For example, in epitope regions that contain an alpha helix or a beta sheet structure, the replacement amino acids in the analogous sequence preferably maintain the same specific structure.

As used herein, "homologous protein" refers to a protein **(***e.g*., protease) that has similar catalytic action, structure, antigenic, and/or immunogenic response as the protein (*e.g.*, protease) of interest. It is not intended that a homolog and a protein (*e.g.*, protease) of interest be necessarily related evolutionarily. Thus, it is intended that the term encompass the same functional protein(s) obtained from different species. In some preferred embodiments, it is desirable to identify a homolog that has a tertiary and/or primary structure similar to the protein of interest, as replacement for the epitope in the protein of interest with an analogous segment from the homolog will reduce the disruptiveness of the change. Thus, in most cases, closely homologous proteins provide the most desirable sources of epitope substitutions. Alternatively, in some embodiments, it is advantageous to look to human analogs for a given protein.

As used herein, "homologous genes" refers to at least a pair of genes from different, but usually related species, which correspond to each other and which are identical or very similar to each other. The term encompasses genes that are separated by speciation (*i.e*., the development of new species) (*e.g*., orthologous genes), as well as genes that have been separated by genetic duplication (*e.g*., paralogous genes).

As used herein, "ortholog" and "orthologous genes" refer to genes in different species that have evolved from a common ancestral gene (*i.e.,* a homologous gene) by speciation. Typically, orthologs retain the same function in during the course of evolution. Identification of orthologs finds use in the reliable prediction of gene function in newly sequenced genomes.

As used herein, "paralog" and "paralogous genes" refer to genes that are related by duplication within a genome. While orthologs retain the same function through the course of evolution, paralogs evolve new functions, even though some functions are often related to the original one. Examples of paralogous genes include, but are not limited to genes encoding trypsin, chymotrypsin, elastase, and thrombin, which are all serine proteases and occur together within the same species.

The degree of homology between sequences may be determined using any suitable method known in the art (*See e.g.,* Smith and Waterman, Adv. Appl. Math., 2:482 [1981]; Needleman and Wunsch, J. Mol. Biol., 48:443 [1970]; Pearson and Lipman, Proc. Natl. Acad. Sci. USA 85:2444 [1988]; programs such as GAP, BESTFIT, FASTA, and TFASTA in the Wisconsin Genetics Software Package (Genetics Computer Group, Madison, WI); and Devereux et al., Nucl. Acid Res., 12:387-395 [1984]).

For example, PILEUP is a useful program to determine sequence homology levels. PILEUP creates a multiple sequence alignment from a group of related sequences using progressive, pairwise alignments. It can also plot a tree showing the clustering relationships used to create the alignment. PILEUP uses a simplification of the progressive alignment method of Feng and Doolittle (Feng and Doolittle, J. Mol. Evol., 35:351-360 [1987]). The method is similar to that described by Higgins and Sharp (Higgins and Sharp, CABIOS 5:151-153 [1989]). Useful PILEUP parameters including a default gap weight of 3.00, a default gap length weight of 0.10, and weighted end gaps. Another example of a useful algorithm is the BLAST algorithm, described by Altschul *et al.,* (Altschul et al., J. Mol. Biol., 215:403-410, [1990]; and

Karlin et al., Proc. Natl. Acad. Sci. USA 90:5873-5787 [1993]). One particularly useful BLAST program is the WU-BLAST-2 program (*See*, Altschul et al., Meth. Enzymol., 266:460-480 [1996]). Parameters "W," "T," and "X" determine the sensitivity and speed of the alignment. The BLAST program uses as defaults a wordlength (W) of 11, the BLOSUM62 scoring matrix (*See,* Henikoff and Henikoff, Proc. Natl. Acad. Sci. USA 89:10915 [1989]) alignments (B) of 50, expectation (E) of 10, M'5, N'-4, and a comparison of both strands.

As used herein, "percent (%) nucleic acid sequence identity" is defined as the percentage of nucleotide residues in a candidate sequence that is identical with the nucleotide residues of the sequence.

As used herein, the term "hybridization" refers to the process by which a strand of nucleic acid joins with a complementary strand through base pairing, as known in the art.

As used herein, "maximum stringency" refers to the level of hybridization that typically occurs at about Tm-5°C (5°C below the Tm of the probe); "high stringency" at about 5°C to 10°C below Tm; "intermediate stringency" at about 10°C to 20°C below Tm; and "low stringency" at about 20°C to 25°C below Tm. As will be understood by those of skill in the art, a maximum stringency hybridization can be used to identify or detect identical polynucleotide sequences while an intermediate or low stringency hybridization can be used to identify or detect polynucleotide sequence homologs.

In some embodiments, "equivalent residues" are defined by determining homology at the level of tertiary structure for a precursor protein (*e*.g., a protease of interest) whose tertiary structure has been determined by x-ray crystallography. Equivalent residues are defined as those for which the atomic coordinates of two or more of the main chain atoms of a particular amino acid residue of the precursor protein and another protein are within 0.13nm and preferably 0.1nm after alignment. Alignment is achieved after the best model has been oriented and positioned to give the maximum overlap of atomic coordinates of non-hydrogen protein atoms of the protein. In most embodiments, the best model is the crystallographic model giving the lowest R factor for experimental diffraction data at the highest resolution available.

In some embodiments, modification is preferably made to the "precursor DNA sequence" which encodes the amino acid sequence of the precursor protein, but in alternative embodiments, it is made by the manipulation of the precursor protein. In the case of residues which are not conserved, the replacement of one or more amino acids is limited to substitutions which produce a variant which has an amino acid sequence that does not correspond to one found in nature. In preferred embodiments, in the case of conserved residues, such replacements should not result in a naturally-occurring sequence. Derivatives provided by the present invention further include chemical modification(s) that change the characteristics of the protein.

In some preferred embodiments, the protein gene is ligated into an appropriate expression plasmid. The cloned protein gene is then used to transform or transfect a host cell in order to express the protein gene. This plasmid may replicate in hosts in the sense that it contains the well-known elements necessary for plasmid replication or the plasmid may be designed to integrate into the host chromosome. The necessary elements are provided for efficient gene expression (*e.g*., a promoter operably linked to the gene of interest). In some embodiments, these necessary elements are supplied as the gene's own homologous promoter if it is recognized, (*i.e*., transcribed by the host), a transcription terminator (a polyadenylation region for eukaryotic host cells) which is exogenous or is supplied by the endogenous terminator region of the protein gene. In some embodiments, a selection gene such as an antibiotic resistance gene that enables continuous cultural maintenance of plasmid-infected host cells by growth in antimicrobial-containing media is also included.

In some preferred embodiments involving proteases, variant protease activity is determined and compared with the protease of interest by examining the interaction of the protease with various commercial substrates, including, but not limited to casein, keratin, elastin, and collagen. Indeed, it is contemplated that protease activity will be determined by any suitable method known in the art. Exemplary assays to determine protease activity include, but are not limited to, succinyl-Ala-Ala-Pro-Phe-para nitroanilide (SAAPFpNA) (citation) assay; and 2,4,6-trinitrobenzene sulfonate sodium salt (TNBS) assay. In the SAAPFpNA assay, proteases cleave the bond between the peptide and p-nitroaniline to give a visible yellow color absorbing at 405 nm. In the TNBS color reaction method, the assay measures the enzymatic hydrolysis of the substrate into polypeptides containing free amino groups. These amino groups react with TNBS to form a yellow colored complex. Thus, the more deeply colored the reaction, the more activity is measured. The yellow color can be determined by various analyzers or spectrophotometers known in the art.

Other characteristics of the variant proteases can be determined by methods known to those skilled in the art. Exemplary characteristics include, but are not limited to thermal stability, alkaline stability, and stability of the particular protease in various substrate or buffer solutions or product formulations.

For example, alkaline stability can be measured by any suitable method known to those in the art. In preferred embodiments, a substantial change in alkaline stability is evidenced by at least about a 5% or greater increase or decrease (in most embodiments, it is preferably an increase) in the half-life of the enzymatic activity of a mutant when compared to the precursor protease.

In addition, thermal stability can be measured by any suitable method known to those in the art. In preferred embodiments, a substantial change in thermal stability is evidenced by at least about a 5% or greater increase or decrease (in most embodiments, it is preferably an increase) in the half-life of the catalytic activity of a mutant when exposed to a relatively high temperature and neutral pH as compared to the precursor protease.

### DETAILED DESCRIPTION OF THE INVENTION

The present invention provides means to assess the comparative allergenicity of proteases. In particular, the present invention provides means to qualitatively assess the potential for any protease to induce an allergic response in humans. In addition, the present invention provides means to select proteases with reduced allergenicity for use in various applications.

Proteolytic activity by common aeroallergens has been described as facilitating Th2 responses. Proteolytic allergens such as Der p1, remove immunomodulatory cell-surface molecules from the surface of T and B cells. Industrial proteases are known to both induce and potentiate allergic responses. In addition, industrial proteases demonstrate differential allergenic potencies in occupationally exposed workers. As described in greater detail herein, the effects of three industrial proteases, *B. lentus* subtilisin, BPN' Y217L, and *B. licheniformis* subtilisin on cell surface markers and human cytokines were investigated to address potential mechanisms of allergy induction and assess their different potencies.

As indicated herein, the proteases were found to have similar effects on cell surface markers, including removal of CD25, CD8 and CD4. The proteases also were found to display significant specificity for human IL-10 (*i.e*., interleukin-10), TGF-β (*i.e*., transforming growth factor beta), and IL-13, with IC50 values in the ng/ml range. Although it is not intended that the present invention be limited to any particular mechanism(s), it is contemplated that at least one explanation of the differential allergenic potency observed is due to the activity on IL-4. Indeed, it is contemplated that the activity of these proteases on regulatory cell associated cytokines explains their overall allergenicity. As indicated above, although it is not intended that the present invention be limited to any particular mechanism(s), it is contemplated that proteolytic activity directed toward cytokines provides at least one general mechanism of allergic induction.

The activation of CD4+ T-cells along their differentiation pathways results from antigen-specific signaling by antigen-presenting cells. The factors that control the differentiative outcome of antigen signaling are complex, but include cytokine-mediated signaling. The cytokines IL-4 and IL-12 are involved in instructing naïve CD4+ T-cells to become Th2 and Th1 cells, respectively. IL-4 is an absolute requirement for the development of Th2 responses (*See e.g.,* Le Gros et al., J. Exp. Med., 172:921 [1990]). Another outcome of T-cell activation is the induction of non-responsiveness. There are a number of mechanisms whereby tolerance to antigens is believed to become established, including deletional mechanisms active both in the thymus during development and in the periphery in response to exogenous antigen, clonal "ignorance" (*i.e*., where T-cells with specificity for antigen do not respond to sequestered antigens), and active suppression of responses by regulatory T-cells. The differentiation of regulatory T-cells may also be influenced by cytokine signaling as TGF-beta and IL-10, have been shown to be necessary for their induction (*See e.g.,* Chen et al., J. Exp. Med., 198:1875; and Levings et al., Blood 105:1162 [2005].

IgE-mediated hypersensitivity reactions are a result of a subset of immune responses characterized by the preferential differentiation of Th2 CD4+ T-cells. The induction of allergic responses tends to occur at mucosal surfaces (*e.g*., within the lungs and the gut). Although it is not intended that the present invention be limited to any particular mechanism(s), regulatory T-cells in the lung and the gut are believed to control allergic type responses in non-allergic individuals. Thus, the disregulation of antigen-specific responses is believed to result in allergies in some individuals. The properties of allergens that sway immune responses from the normal non-allergic responses to commonly encountered proteins to a Th2-type of response are not completely understood. However, many allergens, including many respiratory allergens, have been described to possess proteolytic activity. For example, pollen allergens are a complex mixture of many proteins including a variety of peptidases, and many recombinant allergen proteins also display proteolytic activity (*See e.g.,* Hewitt et al., Allergy 53:60 [1998]; and Bagarozzi et al., Phytochem., 47:593 [1998]).

In some experiments, proteolytic activity was found to be necessary to induce allergic responses, as inactivated proteases were not allergenic (*See e.g.,* Shakib et al., Clin. Exp. Allergy 30:751 [2000]; Pollock et al., J. Immunol., 170:1746 [2002]; and Kheradmand et al., J. Immunol., 169:5904 [2002]). Co-administration of a proteolytic allergen with a normally non-allergenic protein was found to induce IgE responses to the second protein, demonstrating that the proteolytic activity was acting in trans. The mechanism by which the proteolytic activity was affecting the immune response was described as being contributed by an effect on cell-surface regulatory molecules.

As indicated previously, the manufacture of industrial proteases has been shown to induce sensitization in exposed workers (*See e.g.,* Cullinan *et al., supra;* Novey *et al., supra;* Pepys *et al., supra;* Vanhanen *et al., supra;* and Schweigert *et al., supra*). Also as indicated above, the rates of sensitization range from 3 to 11 percent per year, depending on the material being processed (*See e.g.,* Sarlo and Kirchner, *supra;* and Sarlo *et al.,* Fund. Appl. Toxicol., supra). However, two highly related proteases, *B. lentus* subtilisin and BPN' Y217L, induce distinctly different rates of sensitization in exposed industrial workers. Implementation of improved work practices and control measures has reduced the number of allergy cases among occupationally exposed workers (*See e.g.,* Sarlo and Kirchner, *supra;* and Sarlo, *supra*). The quantities of protease encountered during occupational exposure are inconsistent with the level of enzyme necessary *in vitro* and *in vivo* to exert effects on cell surface markers and to initiate Th2 responses. Thus, during the development of the present invention, other factors that control Th cell differentiation and are exquisitely sensitive to proteolysis were investigated. In order to assess the differences between the potencies of two related industrial proteases, various experiments were conducted to determine the proteolytic activity of various industrial proteases on human cytokines were developed and tested.

The results obtained during the development of the present invention indicated that human cytokines exhibit differential sensitivities to serine protease allergens. Cytokines associated with regulatory T-cells were the most sensitive to proteolysis by all three tested proteases. IC50 values for IL-10, IL-13 and TGF-β were less than 100 ng/ml. In contrast, cytokines associated with the establishment of Th1 responses, IL-12 and IFN-γ, were comparatively less sensitive, with IC50s in the ug/ml range. Interestingly, the Th2 cytokine IL-4 may be an indicator of allergenic potency as a comparatively less potent allergen, BPN' Y217L, displayed significantly more activity on IL-4 than two other related serine proteases. However, it is not intended that the present invention be limited to these particular cytokines or proteases, alone or in any combination.

IL-10 and TGF-β are cytokines that are associated with both the differentiation and the effector function of regulatory T-cells in both mice and humans (*See,* Chen *et al., supra;* Groux et al., Nature 389:737 [1997]; Yamagiwa et al., J. Immunol., 166:7282 [2001]; Read et al., J. Exp. Med., 192:295 [2000]; and Akbari et al., Nat. Immunol., 2:725 [2001]). As regulatory T-cells are believed to control allergic-type responses in non-allergic individuals, the allergenic property of the tested proteases is contemplated to result from the reduction in the levels of immunomodulatory cytokines IL-10 and/or TGF-β, mediated by inhibition of the differentiation of regulatory cells in response to protein antigens, and/or by interference with suppressive activity by pre-existing regulatory T-cells (*See,* Taylor et al., Int. Arch. Allergy Immunol., 135:73 [2004]; and Akdis et al., J. Exp. Med., 199:567 [2004]). However, it is not intended that the present invention be limited to any particular mechanism(s).

The protease effect on IL-13 is of interest, as this cytokine has been described as contributing to the development of allergic responses (*See*, Herrick et al., J. Immunol., 170:2488 [2003]). Therefore, loss of this cytokine in the microenvironment where allergen-specific responses are occurring is contemplated to reduce allergic responses. However, it is also contemplated that IL-13 has a unique role in the development of allergic asthma, as IL-13 has been shown to induce airway hyper-reactivity (AHR) and mucus secretion independently of IgE induction and eosinophilia (*See,* Grunig et al., Science 282:2261 [1998]; Wills-Karp et al., Science 282:2258 [1998]; and Ford et al., J. Immunol., 167:1769 [2001]). Specific reduction of IL-13 levels in the presence of normal levels of IL-4 may result in the induction of IgE-mediated allergic responses without AHR and mucus secretion typical of asthma. While mouse intranasal tests of these enzymes have been developed, airway hyper-reactivity was not a measured outcome (*See,* Robinson et al., Fund. Appl. Toxicol., 34:15 [1996]). Other mouse intranasal tests of *Aspergillus* proteolytic enzymes did show the induction of AHR (*See,* Kherammand *et al., supra*). It is contemplated that these particular enzymes induce a unique type of allergic response unlinked from the development of AHR. However, as indicated previously, it is not intended that the present invention be limited to any particular mechanism(s).

It is further contemplated that other regulatory mechanisms are also affected by protease activity. For example, CD8+ suppressor cells have been described and have been shown recently to have a down-regulatory effect in a mouse model of allergic inflammation (*See e.g.,* Stock et al., Eur. J. Immunol., 34:1817 [2004]). As described herein, cell surface CD8 was found to be exquisitely sensitive to enzymes, another mechanism whereby it is contemplated that enzymes modify suppressive responses. The IL-2 receptor alpha chain (CD25), is a marker for both activated T-cell and in conjunction with CD4 and *foxp3* expression is a marker of regulatory T-cells. Both *B. lentus* subtilisin and BPN' Y217L remove CD25 from the surface of PHA-induced PBMC blasts at very low concentrations of enzyme. It is contemplated that the loss of cell surface CD25 influences subsequent activation of T-cells. Indeed, this has been proposed as a mechanism whereby some other proteolytic allergens mediate their effect (*See e.g.,* Schultz et al., J. Exp. Med., 187:271 [1998]; and Shakib et al., Immunol. Today 19:313 [1998]). Two of the enzymes described herein have distinct allergenic potencies in exposed occupational workers, with *B*. *lentus* subtilisin being stronger than BPN' Y217L (*See e.g.,* Robinson *et al., supra*). However, the overall affect on the cell surface markers, as described herein, including CD25, was not different. The only significant difference noted was in the proteolytic specificity for IL-4, where the less allergenic protease displayed a significantly lower IC50 value for IL-4. This result indicated that the more potent allergen, *B. lentus* subtilisin, .. mediates its effect by inhibiting regulatory activity by reducing local concentrations of IL-10 and TGF-β, leaving the levels of IL-4 in the microenvironment comparatively untouched. This is contemplated to support the development of a Th2-biased immune response. However, as above, it is not intended that the present invention be limited to any particular mechanism(s).

It is contemplated that the effect of proteases on cytokines are relevant, as the concentrations required to produce effects are very small. In contrast to the published effects of proteolytic allergen molecules on cell surface marker expression (5-10 ug/ml for CD25 removal (*See,* Shakib *et al., supra*), the results described for the proteolytic enzymes herein displayed IC50 values against cytokines at pg/ml levels. As all of the assays were performed in media containing 5% human serum, significant specificity for the cytokine molecules was displayed. Exposure to these proteases in an industrial setting is very low, inconsistent with the concentrations of other proteases needed to observe *in vitro* effects, but they are capable of promoting allergic type responses *in vivo.* For example, Der p 1 causes skewing to Th2 and IgE at 10 ug/mouse as an *in vivo* dose, which is in variance with the concentrations needed to show biological effects *in vitro* (*See,* Gough et al., J. Exp. Med., 190:1897 [1999]). In a similar *in vivo* model, *Aspergillus fumigatus* proteases were active in promoting Th2 responses at a 33 ug/dose in mice (*See*, Kheradmand *et al., supra*).

Many allergens have been characterized as possessing proteolytic activity (*See e.g.,* Hewitt et al., Allergy 53:60 [1998]; Stewart et al., Curr. Opin. Allergy Clin. Immunol., 1:95 [2001]; and Widmer et al., Clin. Exp. Allergy 30:571 [2000]). In addition, pollen granules are comprised of a complex mixture of proteins including serine endopeptidases with activity on immunomodulatory peptides such as substance P and angiotensin 1 and 2 (*See e.g.,* Bagarozzi et al., Phytochem., 47:593 [1998]; and Bagarozzi et al., Am. J. Resp. Cell Mol. Biol., 18:363 [1998]). If proteolytic activity on immunomodulatory cytokines results in the inactivation of regulatory cell activity and subsequent development of allergic type responses, it follows that immune responses to bystander antigens will also be affected. This effect could explain the potentiating effect of proteases on non-allergenic proteins (*See e.g.,* Kheradmand *et al., supra;* Kurup et al., Int. Arch. Allergy Immunol., 129:129 [2002]; and Sarlo et al., J. Allergy Clin. Immunol., 100:480 [1997]). It is also contemplated that this provides one explanation for the observation that allergic donors are rarely allergic to a single protein.

### EXPERIMENTAL

The following examples serve to illustrate certain preferred embodiments and aspects of the present invention and are not to be construed as limiting the scope thereof.

In the experimental disclosure which follows, the following abbreviations apply: eq (equivalents); M (Molar); µM (micromolar); N (Normal); mol (moles); mmol (millimoles); µmol (micromoles); nmol (nanomoles); g (grams); mg (milligrams); kg (kilograms); µg (micrograms); L (liters); ml (milliliters); µl (microliters); cm (centimeters); mm (millimeters); µm (micrometers); nm (nanometers); °C. (degrees Centigrade); h (hours); min (minutes); sec (seconds); msec (milliseconds); xg (times gravity); Ci (Curies); OD (optical density); Dulbecco's phosphate buffered solution (DPBS); HEPES (N-[2-Hydroxyethyl]piperazine-N-[2-ethanesulfonic acid]); HBS (HEPES buffered saline); SDS (sodium dodecylsulfate); Tris-HCl (tris[Hydroxymethyl]aminomethane-hydrochloride); IC50 (inhibitory concentration of 50%); Klenow (DNA polymerase I large (Klenow) fragment); rpm (revolutions per minute); EGTA (ethylene glycol-bis(β-aminoethyl ether) N, N, N', N'-tetraacetic acid); EDTA (ethylenediaminetetracetic acid); SPT+ (skin prick test positive); ATCC (American Type Culture Collection, Rockville, MD); BDIS (Becton-Dickinson Immunocytometry Systems, San Jose, CA); BenderMed Systems (BenderMed Systems, Vienna, Austria); Cedar Lane (Cedar Lane Laboratories, Ontario, Canada); Gibco and Gibco/Life Technologies (Gibco/Life Technologies, Grand Island , NY); Sigma (Sigma Chemical Co., St. Louis, MO); Pharmacia (Pharmacia Biotech, Piscataway, NJ); Perseptive (Perseptive Biosystems, Framingham, MA); Procter & Gamble (Procter and Gamble, Cincinnati, OH); Genencor (Genencor International, Palo Alto, CA); Endogen (Endogen, Woburn, MA); Cedarlane (Cedarlane, Toronto, Canada); Dynal (Dynal, Norway); Novo (Novo Industries A/S, Copenhagen, Denmark); Biosynthesis (Biosynthesis, Louisville, TX); R&D Systems (R&D Systems, Inc., Minneapolis, MN); TriLux Beta, (TriLux Beta, Wallac, Finland); Wallac (Wallac, Turku, Finland); DuPont/NEN (DuPont/NEN Research Products, Boston, MA); TomTec (Hamden, CT); and Stratagene (Stratagene, La Jolla, CA).

In the following Examples, the following proteases were used. *B. lentus* subtilisin (Swissprot accession number P29600), BPN' Y217L (Swissprot accession number P00782), *B*. *licheniformis* subtilisin ("Carlsberg"; Accession number AAU40017), LG-12 (Bacillus sp. Accession number U39230) and BPN' Y217L N155G ("N155G"; this protease is a variant of BPN' Y217L with an amino acid substitute that reduces its proteolytic activity on the synthetic substrate succ-pAAPF to 0.1% of the parent enzyme) were purified from culture broths utilizing the BioCad 700E system (Perseptive) and POROS HS/M resin (Perseptive) on porous polystyrene beads, and was provided for use at approximately 45 mg/mL in 20 mM MES pH 5.8, 1 mM CaCl₂. Aliquots of subtilisin were kept frozen at -20°C until use.

### ELISA Experiments

ELISA assays were used to determine cytokine activity following exposure to the test enzymes. In these experiments, purified recombinant cytokines were purchased from R & D Systems. Cytokines were co-cultured with active enzymes in 5% human AB serum-containing RPMI-1640 (Gibco) at 37°C for 12-18 hours. Enzymatic activity was stopped by the addition of 1 mM PMSF. ELISAs were performed using DuoSet ELISA kits (R and D Systems) for IL-2, IL-10, IL-12p70, and TGF-β1. Instant ELISA kits (BenderMed Systems) were used to measure IL-4 and IFN-γ. All kits were used according to the manufacturers' recommendations. IC50 values were calculated from regression trendlines calculated using the linear portion of the curves and solving the slope equation for the 50% value.

### Cell Surface Cleavage Assays

Peripheral blood mononuclear cells (PBMC) were prepared from donor buffy coats purchased from the Stanford Blood Center (Palo Alto, CA). Buffy coat material was diluted in DPBS and separated on a discontinuous Lymphopaque gradient (Gibco). PBMC were resuspended to 10⁶ cells/ml in DPBS (Gibco), then incubated with varying concentrations of subtilisin at room temperature for one hour. The reactions were stopped by the addition of 2% FCS (Sigma) in DPBS and by repeated washings. After enzyme treatment, cells were resuspended in DPBS and incubated with fluorescent antibodies for 30 minutes at room temperature. Cells were washed in DPBS, then analyzed on a FACSCalibur flow cytometer (BDIS) and the data analyzed using CellQuest software (BDIS).

### Functional Assays

TF-1 cells (CRL-2003) were purchased from ATCC. After overnight incubation of IL-4 with proteases, PMSF was added to 1 mM final concentration. TF-1 cells were washed three times and cultured at 2 x 10⁴ cells per well in a 96 well plate with the enzyme-treated cytokines. Cultures were incubated at 37°C in 5% CO₂ for 48 hours. Then, 0.5 uCi of tritiated thymidine was added to each well. Cultures were harvested 18 hours later and processed for scintillation counting using a Tri-Lux Scintillation counter (Wallac).

### COMPARATIVE EXAMPLE 1

### Cell Surface Markers

These experiments were conducted to determine whether removal of cell surface molecules by proteases influences the type of subsequent immune response. In particular, as the proteases described herein have different allergenic potencies, their effect on relevant surface markers were investigated, in order to determine whether they are distinguishable. Human PBMC were treated with *B. lentus* subtilisin and BPN' Y217L. After treatment, the presence of cell surface markers was detected using flow cytometry (*See,* Figure 3). Neither enzyme removed HLA-DR, or CD86 from the surface of human monocytes. In fact, enzyme treatment resulted in a slight but reproducible upregulation of CD86 and HLA-DR. Also, the enzymes had no effect on CD3 expression. *B. lentus* subtilisin and BPN' Y217L have identical activity on the expression of CD8 and CD25. However, BPN' Y217L treatment resulted in lower levels of expression of CD4 at higher doses, and a lower IC50. The IC50 values for both CD4 and CD8 removal from the surface of PBMC were in the ug/ml range. Thus, the results indicated that cell surface markers are removed similarly by both BPN' Y217L and *B. lentus* subtilisin.

### EXAMPLE 2

### Activity of Serine Proteases on Human Cytokines

In these experiments, designed to determine the activity of serine proteases on human cytokines, BPN' Y217L and *B. lentus* subtilisin were incubated overnight at 37°C with recombinant human cytokines in 5% human serum. As a control, the BPN' Y217L variant N155G was also tested. N155G was found to exhibit 0.1% of the control enzyme activity on the tetrapeptide substrate succ-pAAPF. After incubation, protease activity was inhibited by the addition of PMSF to a final concentration of 1 mM. The residual cytokine levels were tested in ELISA assays. Figure 2 provides graphs showing the results for IL-4, IL-13, IL-12 and IL-10.

BPN' Y217L and *B. lentus* subtilisin were found to have equivalent activity on all the cytokines tested except IL-4. There was a marked difference between the BPN' Y217L and subtilisin in the levels of enzyme needed to reduce the detectable levels of IL-4. BPN' Y217L was approximately 60-fold more active, as indicated in Table 1, below. These data represent results from two experiments, where indicated. In this Table, "ND" indicates "not done." The enzymes were active on IL-13 in the ng/ml range, as opposed to IL-10, for which activity was observed in the ug/ml dose range. In sum, the results obtained in these experiments indicated that serine proteases are active on human cytokines at very low concentrations *in vitro.*

| **Table 1. IC50 Values (ug/ml) of Proteolytic Enzymes on Human Cytokines** | | | | | |
|---|---|---|---|---|---|
| **Cytokine** | **Protease** | | | | |
| | **BPN' Y217L** | ***B. lentus* Subtilisin** | **Carlsberg** | **LG12** | **N155G** |
| **IL-4** | <0.02, 0.04 | 1.25, 1.5 | 1.25 | 0.25 | 7.5 |
| **IL-10** | 0.04, 0.04 | 0.1, 0.11 | 0.23 | 0.02 | 2 |
| **IL-12 p70** | 2,3.75 | 3.5, 1.9 | 2.5 | 0.5 | >10 |
| **IL-13** | 0.01, 0.005 | 0.01,0.005 | 0.05 | 0.005 | 8 |
| **IFN-γ** | 2.5, 3.75 | 1.25, 4.0 | 6 | 0.88 | ND |
| **TGFβ1** | 0.09,0.01 | 0.11, 0.04 | 0.22 | 0.18 | ND |

The results obtained from these experiments also showed that a less potent allergen has comparatively more activity on IL-4. As indicated above, the activity of four different serine protease allergens on human cytokines was *tested. B. licheniformis* protease Carlsberg and *B*. *lentus* subtilisin have equivalent activity in animal models of allergenicity, and induce approximately the same rate of sensitizations in occupationally exposed workers (*See e.g.,* Robinson et al., Toxicol. Sci., 43:39 [1998]).

BPN' Y217L is less active in animal models, possessing about 0.3x the potency as compared to Carlsberg. BPN' Y217L also demonstrates a lower rate of sensitization in humans than Carlsberg or *B. lentus* subtilisin. Both *B. lentus* subtilisin and Carlsberg displayed equivalent levels of activity on the tested cytokines, consistent with their largely similar allergenic potencies (*See,* Figure 3). The IC50 values were in the ug/ml range for IL-12, and were less than 200 ng/ml for IL-10 and TGF-beta. The exception was human IL-4, where *B*. *lentus* subtilisin and Carlsberg displayed substantively less activity as compared to BPN' Y217L.

The LG-12 protease was overall more active on all human cytokines. However, it would be predicted to have an allergenic potential intermediate between B. lentus subtilisin and BPN 'Y217L due to its demonstrated activity of IL-4. There is no human exposure data for LG-12.

As indicated in Table I, activity of proteases on human cytokines falls into two general categories: IC50s in the ug/ml range, and IC50s in the ng/ml range. The cytokines comparatively resistant to serine protease cleavage were found to be IL-12 and IFN-gamma. IL-4 is resistant to *B. lentus* subtilisin and Carlsberg. Sensitive cytokines were found to include IL-13, IL-10 and TGF-beta. IC50 values for the sensitive cytokines were determined to be less than 100 ng/ml.

As the enzymes involved in these experiments are known to be highly active serine proteases, and could potentially be active on ELISA assay components, three of the proteases used in these experiments used were tested and found not to detectably affect the mouse IgG1 ELISA plate coating reagents used in most of the assays. It was contemplated that proteolytic activity on the cytokines tested destroys their ability to be detected in the ELISA assays but not affect their biological function. To test this, human IL-4 was treated with the proteases in an identical assay format as utilized in the ELISA assays shown in Figures 2 and 3. After incubation and inhibition of the enzymes with PMSF, IL-4 responsive TF-1 cells were added to the wells. Proliferation of the TF-1 cells was detected on day 3 (*See,* Figure 4). The reduction in proliferation by the TF-1 cells corresponds with the loss of detection of IL-4 in the ELISA assays. Thus, proteolytic activity reduces the functional activity of human IL-4.

All patents and publications mentioned in the specification are indicative of the levels of those skilled in the art to which the invention pertains.

Having described the preferred embodiments of the present invention, it will appear to those ordinarily skilled in the art that various modifications may be made to the disclosed embodiments within the scope of the present invention.

Those of skill in the art readily appreciate that the present invention is well adapted to carry out the objects and obtain the ends and advantages mentioned, as well as those inherent therein. The compositions and methods described herein are representative of preferred embodiments, are exemplary, and are not intended as limitations on the scope of the invention.

## Claims

1. An in vitro method for assessing the potential of a protease to induce an allergic response in humans comprising exposing a cytokine selected from the group consisting of interleukins, interferons and transforming growth factors to said protease, and comparing the activity of said cytokine before and after exposure to said protease.

2. A method according to claim 1 wherein the cytokine is a human cytokine.

3. A method according to claim 2 wherein the cytokine is selected from the group consisting of IL-4, IL-13, IL-12, TGF-β and IL-10.

4. A method according to any one of claims 1 to 3 wherein the protease is a serine protease.

5. A method according to claim 4 wherein the serine protease is a subtilisin.

6. A method according to claim 5 wherein the subtilisin is selected from the group consisting of *B. lentus* subtilisin, *B. amyloliquefaciens* subtilisin (BPN'), and variants of said *B*. *lentus* and *B. amyloliquefaciens* subtilisins.

7. A method according to any one of the preceding claims wherein the activity of said cytokine is determined by ELISA.

## Patentansprüche

1. In-vitro-Verfahren zur Beurteilung des Potenzials einer Protease zum Induzieren einer allergischen Reaktion in Menschen, umfassend, ein Zytokin, ausgewählt aus der Gruppe, bestehend aus Interleukinen, Interferonen und transformierenden Wachstumsfaktoren, der Protease auszusetzen und die Aktivität des Zytokins vor und nach dem der Protease Ausgesetztsein zu vergleichen.

2. Verfahren nach Anspruch 1, wobei das Zytokin ein menschliches Zytokin ist.

3. Verfahren nach Anspruch 2, wobei das Zytokin aus der Gruppe ausgewählt ist, bestehend aus IL-4, IL-13, IL-12, TGF-β und IL-10.

4. Verfahren nach einem der Ansprüche 1 bis 3, wobei die Protease eine Serinprotease ist.

5. Verfahren nach Anspruch 4, wobei die Serinprotease ein Subtilisin ist.

6. Verfahren nach Anspruch 5, wobei das Subtilisin aus der Gruppe ausgewählt ist, bestehend aus *B. lentus*-Subtilisin, *B. amyloliquefaciens-Subtilisin* (BPN') und Varianten der *B. lentus-* und *B. amyloliquefaciens-Subtilisine.*

7. Verfahren nach einem der vorstehenden Ansprüche, wobei die Aktivität des Zytokins durch ELISA bestimmt wird.

## Revendications

1. Méthode in vitro pour l'évaluation du potentiel d'une protéase à induire une réponse allergique chez des humains comprenant l'exposition d'une cytokine choisie dans le groupe constitué d'interleukines, d'interférons et de facteurs de croissance transformants à ladite protéase, et la comparaison de l'activité de ladite cytokine avant et après l'exposition à ladite protéase.

2. Méthode selon la revendication 1, dans laquelle la cytokine est une cytokine humaine.

3. Méthode selon la revendication 2, dans laquelle la cytokine est choisie dans le groupe constitué de IL-4, IL-13, IL-12, TGF-β et IL-10.

4. Méthode selon l'une quelconque des revendications 1 à 3, dans laquelle la protéase est une sérine protéase.

5. Méthode selon la revendication 4, dans laquelle la sérine protéase est une subtilisine.

6. Méthode selon la revendication 5, dans laquelle la subtilisine est choisie dans le groupe constitué de: subtilisine de *B. lentus,* subtilisine de *B. amyloliquefaciens* (BPN') et variantes desdites subtilisines de *B. lentus* et de *B. amyloliquefaciens.*

7. Méthode selon l'une quelconque des revendications précédentes, dans laquelle l'activité de ladite cytokine est déterminée par ELISA.
